## Europäisches Patentamt
## European Patent Office
## Office européen des brevets

(11) Numéro de publication: **0 046 614**
**B1**

(12) **FASCICULE DE BREVET EUROPEEN**

(45) Date de publication du fascicule du brevet:
01.08.84

(51) Int. Cl.³: **C 12 N 11/00**

(21) Numéro de dépôt: **81200881.1**

(22) Date de dépôt: **05.08.81**

(54) Procédé d'immobilisation de cellules microbiennes globulaires par adhésion à un support solide.

(30) Priorité: **22.08.80 BE 201827**

(43) Date de publication de la demande:
**03.03.82 Bulletin 82/9**

(45) Mention de la délivrance du brevet:
**01.08.84 Bulletin 84/31**

(84) Etats contractants désignés:
**AT CH DE FR GB IT LI LU NL SE**

(56) Documents cités:
**BE - A - 884 878**
**FR - A - 2 180 005**
**GB - A - 2 001 654**
**GB - A - 2 022 595**
**US - A - 3 115 404**
**US - A - 3 821 086**
**US - A - 3 841 969**

**CHEMICAL ABSTRACTS, vol. 87, no. 11, 12 septembre 1977 page 260, abrégé 80837n Columbus, Ohio, US J.J. MONAHAN "Magnesium acetate treatment of glass roller bottles to facilitate cell attachment"**
**CHEMICAL ABSTRACTS, vol. 90, no. 23, 4 juin 1979 page 501, abrégé 184935w Columbus, Ohio, US**
**CHEMICAL ABSTRACTS, vol. 87, no. 1, 4 juillet 1977 page 334, abrégé 4003x Columbus, Ohio, US I.**

(73) Titulaire: **Région Wallonne représentée par l'Exécutif Régional Wallon, 11, Boulevard de l'Empereur, B-1000 Bruxelles (BE)**

(72) Inventeur: **Rouxhet, Paul Gérard Sciences Agronomiques, Unité de Physico-Chimie Place de l'Université 1, B-1348 Louvain-La-Neuve (BE)**

(74) Mandataire: **Blanchart, André, Cellule de Gestion des Contrats Technologiques Place Joséphine Charlotte 3 (Boîtes 27, 28), B-5100 Namur (BE)**

(56) Documents cités: (suite)
**ALEMZADEH et al. "Bacterial flocculation with sodium bentonite"**
**JOURNAL INSTIT. BREWING vol. 84, no. 4, 1978 London, GB F.H. WHITE et al. "Continuous fermentation by immobilized brewers jeast", pages 228-230**
**NATURE, vol. 235, 18 février 1972 Basingstoke, GB J.S. HOUGH et al. "Couplings of enzymes onto microorganisms" page 389**

Le dossier contient des informations techniques présentées postérieurement au dépôt de la demande et ne figurant pas dans le présent fascicule.

## Description

La présente invention a pour objet un procédé d'immobilisation de cellules microbiennes globulaires sous forme d'une couche régulière sur un support solide, par l'adsorption d'ions métalliques sur le support solide et/ou les cellules microbiennes.

L'invention a également pour objet la couche de cellules microbiennes ainsi obtenues.

On connait déjà des procédés d'immobilisation de cellules microbiennes, faisant appel à la fixation sur un support, à la formation de flocons ou àl'inclusion dans une matrice. L'immobilisation par fixation, également appelée adsorption ou adhésion, sur un support solide présente par rapport aux autres procédés, l'avantage de maintenir un contact direct de l'ensemble de la population microbienne avec le milie liquide et, en conséquence, de réduire la limitation, par les phénomènes diffusionnels, du transport des substances nutritives ou des substances produites par les microorganismes. Elle présente également l'avantage de fournir un système dont la géométrie ne peut se modifier au cours de l'utilisation.

Le procédé faisant l'objet du brevet U.S.A. 3.821.o86 permet la formation de flocons de cellules microbiennes sous l'action de polyélectrolytes. Le procédé du brevet belge 804.502 concerne l'obtention de flocons par la formation de chélates entre les cellules microbiennes et des hydroxydes métalliques.

Ces procédés présentent l'inconvénient de ne pas maintenir un contact direct de l'ensemble de la population microbienne immobilisée avec le milieu liquide.

Le procédé décrit dans le brevet U.S.A. 4.115.198, visant principalement l'immobilisation d'enzymes mais mentionnant la posibilité d'immobiliser des cellules entières, fait appel à un recouvrement d'un support par précipitation sur ce dernier d'un oxyde métallique hydraté. La fixation des substances biologiquement actives est attribuée à la formation de chélates.

Ce procédé présente l'inconvénient de former nécessairement des précipités, ce qui exige un contrôle des caractéristiques de ces précipités.

Selon le document CA.87,260n° (John J. Monahan), le support (verre) traité avec une solution d'acétate de Mg retient les cellules pour autant qu'il y ait eu une opération de séchage et d'autoclave.

Il est connu également de traiter par des métaux de transition un support organique, ensuite de le sécher et de le mettre en contact en milieu agité avec les cellules. (Ann. N.Y. Acad. Sci. 1979, 326, 249–54). Ce procédé présente l'inconvénient d'être limité à un support organique, d'exiger un séchage, qui est une opération lonque et coûteuse, et de ne pas premettre un recouvrement régulier de la surface du support.

Un des objets de la présente invention est de développer un procédé d'immobilisation des cellules microbiennes globulaires par un procédé simple et efficace ne nécessitant pas de modes opératoires compliqués.

La présente invention, basée sur l'adsorption d'ions métalliques sur les cellules microbiennes et/ou le support solide, a pour but de fixer une couche cellulaire unique, distribuée régulièrement sur toute la surface du support et y adhérant fortement.

Les cellules immobilisées suivant le procédé de la présente invention présentent un degré de viabilité éléve, c'é-à-dire qu'une Proportion d'entre elles conserve la capacité de réaliser notamment des réactions enzymatiques et de se reproduire dans un milieu approprié.

Un autre but de l'invention est d'obtenir des complexes stables formés d'une couche cellulaire unique et du support pouvant être utilisés directement dans des réacteurs, par exemple de fermentation. Par complexes stables, on comprend également des complexes de nature différente (support et/ou cellule) pouvant par exemple être combinés et utilisés sous forme de couches lamellaires multiples. On peut par exemple, superposer sur un même support des couches cellulaires immobilisées de différents microorganismes, ou superposer une série de complexes constitués chacun d'un support auquel est fixée une couche celulaire, les microorganismes étant de nature identique ou différente d'une couche à l'autre ou au sein de la même couche.

L'invention, telle que caractérisée dans les revendications, consiste à traiter, préalablement à l'immobilisation, les celules et/ou le support, par des solutions contenant des ions métalliques simples et/ou des ions métalliques polynucléaires formés par association de plusiuers atomes métalliques, d'oxygène et d'hydrogène. Ces ions simples et/ou polynucléaires s'adsorbent sur les matériaux traités.

Le lien entre les cellules et le support s'explique par une résultante favorable des interactions aux interfaces cellule-solutions, support-solution et cellule-support. Parmi ces interactions, on peut distinguer les interactions électrostatiques (charge-charge et charge-dipôle), les interactions de Van der Waals, les liasisons hydrogène. Etant donné l'importance des interactions électrostatiques charge-charge, le fait que les ions adsorbés diminuent la charge du support ou de la cellule, ou rendent la charge de l'un de signe contraire à la charge de l'autre est un élément très favorable.

Pour expliquer le comportement des systèmes et le choix des conditions opératoires, il faut également tenir compte des propriétés chimiques de ces ions en fonction du pH.

Les support solides utilisés suivant la présente invention cmprennent des matériaux solides minéraux et organiques. Le support à utiliser peut être poreux ou non; pour certaines applications il peut être préférable d'utiliser un support non poreux et ne présentant pas des alvéoles en surface, de manière à ce que toutes les celllules immobilisées puissent être directement exposées au flux d'un liquide. Parmi les composés minéraux, citons l'utilisation de silice, de silicates et d'aluminosilicates, d'oxydes métalliques, de métaux et de leurs alliages, etc... On peut utiliser par exemple le verre, le laitier, le sable. Parmiles composés organiques, citons l'utilisations de polymères et/ou copolymères synthétiques, par exemple les polyamides, les polyesters, les polyoléfines, les composés vyniliques.

Les composés minéraux et organiques cités ci-avant ne constituent que des exemples de supports pouvent être utilisés suivant la présente invention. Pour le choix du support,on tiendra compte à la fois de considérations physiques et mécaniques, économiques et opérationnelles.

Les supports utilisés peuvent se présenter sous des formes très diverses, par exemple de granules, poudre, billes, plaques, tubes, films, membranes, tissus, tricots, fils, fibres, profilés quelconques.

Par cellules microbiennes globulaires on comprend les microorganismes unicellulaires et les assemblages de microorganismes du type, par exemple, des levures, des bactéries, des micro-champignons.

Par couche cellulaire, on comprend une couche cellulaire unique ou monocouche présentant sur la surface du support approximativement une assise cellulaire, qui peut être caractérisée par un nombre de cellules par unité de surface, désigné par le terme densité de cellules immobilisées.

Les solutions aqueuses contenant des ions métalliques simples ou polynucléaires utilisées suivant la présente invention sont obtenues à partir de sels métalliques, par exemple de chlorure, nitrate, sulfate, de magnésium et d'alcalino-terreux, d'aluminium, de métaux de transitions, notamment Cr, Fe, Co, Ni, Cu, Zn.

Pour obtenir des solutions d'ions métalliques simples, leur concentration doit être assez faible, leur pH assez bas et la température assez basse pour que, dans les conditions opératoires, la réaction des atomes métalliques avec l'oxygène et l'hydrogène ne donne pas lieu à la formation d'ions polynucléaires ou de précipité. Les ions métalliques polynucléaires sont formés par association de plusieurs atomes métalliques identiques ou différents, d'oxygène, d'hydrogène et éventuellement d'autres éléments.

Pour obtenir des solutions d'ions métalliques polynucléaires, la concentration, le pH, la température et l'âge des solutions doivent être tels que la réaction des atomes métalliques avec l'oxygène et l'hydroxyle, et d'autres anions éventuellement présents dans la sulution, donne lieu à la formation d'ions polynucléaires mais pas de précipté. Suivant les conditions qu'il recherche, l'homme de l'art peut faire varier la nature du métal, la nature de l'anion, la concentration, le pH,la température, l'âge des solutions et introduire des réactifs , par exemple des complexants, susceptibles d'agir sur les processus chimiques impliquant les éléments métalliques.

Il n'est pas exclu d'utiliser des solutions contenant à la fois des ions métalliques simples et polynucléaires.

Suivant une première forme de réalisation de la présente invention, les cellules microbiennes globulaires sont traitées par des solutions aqueuses contenant des ions métalliques simples. Ce traitement est obtenu par exemple de la manière suivante: On introduit simultanément 10 ml d'une suspension de Sacharomyces cerevisiae à la concentration d'environ $500.10^6$ cellules/ml et 10 ml d'une solution de nitrate d'aluminium de 2 à 200 mg Al/l dans un tube de verre d'une contenance de 30 ml. Le tube bouché est agité pendant 24 h à 25°C. Le pH des suspensions varie de 3,6 à 3,9 suivant leur composition. Les cellulles sont séparées de la solution par centrifugation et l'aluminium restant dans le surnageant est dosé par absorption atomique, la quantité d'aluminium fixé est déterminé par différence.

Les résultats obtenus indiquent que la quantité d'aluminium fixé par les cellules augmente jusqu'à $1,5.10^8$ ions Al par cellule, lorsque la concentration en aluminium dans le surnageant augmente de 0 à 2 mg/l. La quantité d'aluminium fixé reste égale à $1.5.10^8$ ions Al par cellule, lorsque la concentration en aluminium dans le surnageant varie de 2 à 100 mg/l. La fixation de l'aluminium par les cellules répond donc aux caractéristiques d'une adsorption spécifique.

Suivant une variante de réalisation du procédé, les supprts solides sont traités par des solutions contenant des ions métaliques simples. Ce traitement est réalisé par exemple en immergeant le support solide dans une solution aqueuse contenant les ions; le volume et la concentration de la solution sont choisis de manière à absorber une quantité suffisante d'ions à la surface du support.

Suivant une autre variante, on traite à la fois les cellules et le support par des solutions contenant des ions métalliques simples. Dans ce cas, on opère comme ci-dessus pour chacun des traitements et on effectue ensuite l'immobilisation des cellules sur le support.

Suivant une deuxième forme de réalisation de la présente invention, les cellules microbiennes globulaires et/ou le support solide sont traités par des solutions d'ions métalliques polynucléaires. Le traitement est en principe similaire à celui appliqué pour la 1ère forme de réalisation comme indiqué ci-avant.

Il n'est pas exclu d'envisager le traitement des cellules par des solutions contenant des ions métalliques simples et du support solide par des solutions contenant des ions métalliques polynucléaires et inversément. Toutes les formes de combinaison sont possibles.

Suivant cette invention, il n'est pas nécessaire que le support possède des groupements organigues capables de former des chélates avec les ions. Il n'est pas nécessaire de sécher le support après son traitement par les ions. Moyennant une formulation correcte on peut se contenter de traiter les cellules par les ions.

Les avantages obtenus grâce á cette invention sont l'obtention d'une assise unique de cellules toutes fixées au support, d'une quantité élevée de cellules immobilisées par unité de surface du support, d'une distribution régulière des cellules sur la surface du support, d'une forte adhésion des cellules au support. L'immobilisation de cellules en une assise unique et régulière adhérant fortement au support présente l'avantage d'éliminer la diffusion à travers un lit de cellules des substances nutritives et des métabolites produits à proximité de celles-ci, de permettre l'utilisation d'un flux élevé de liquide au contact des cellules et donc une diminution des limitations diffusionnelles, de permettre une régénération homogène de toute la population immobilisée, de permettre l'utilisation des cellules immobilisées sur support aussi bien en lit agité qu'en lit fixe.

Ces avantages sont particulièremt précieux pour certaines utilisations de ces cellules immobilisées, telles que l'assimilation ou la production de substances de poids moléculaire élève, la réalisation d'un dispositif d'ensemencement en continu ou la réalisation d'un dispositif permettant de récolter des cellules de première génération n'ayant pas donné de cellules filles. Le fait de ne pas introduire d'agent chimique autre que le support au contact des cellules et d'utiliser un support chimiquement inerte est un avantage appréciable pour certaines applications des cellules immobilisées.

La présente invention sera mieux comprise à l'aide des exemples ci-après.

Exemple 1

On indroduit simultanément 10 ml d'une suspension de Saccharomyces cerevisiae à la concentration d'environ $500.10^6$ cellules/ml et 10 ml d'une solution aqueuse de nitrate d'aluminium d'une concentration de 2 à 50 mg Al/l dans un tube de verre d'une contenance de 30 ml. Le tube bouché est agité pendant 15 minutes à 25 °C. Le pH de la suspension est inférieur à 3,9; les ions aluminium dissous dans la suspension sont donc des ions simples. Ensuite, on verse la suspension dans un récipient dans lequel se trouve une plaque de verre. La hauteur de la suspension est de 0.5 cm au-dessus du niveau de la plaque. Après environ 5 minutes, la plaque de verre est soumise à un lavage durant 20 minutes par un flux laminaire d'eau d'épaisseur de 4 mm et de vitesse moyenne de 8 cm/sec. Les plaques sont examinées en microscopie optique et les cellules immobilisées sont comptées.

Les cellules immobilsées sont distribuées de manière régulière, formant une couche cellulaire homogène d'une densité de 40 000 cellules/mm². Le test au bleu de méthylène indique que 90 à 99% des cellules immobilisées gardent la capacité de réduire spontanément ce colorant.

Un essai comparatif a été réalisé dans les mêmes conditions que ci-dessus mais sans traitement du support et des cellules par des ions aluminium; les résultats montrent que la densité de cellules immobilisées est inférieure à 600 cellules/mm². Les cellules sont distribuées de manière irrégulière et ne forment pas une couche cellulaire homogène.

Exemple 2

On procède comme décrit dans l'exemple 1 sauf que le support en verre est remplacé par un support en polycarbonate.

On obtient également une couche cellulaire régulière de cellules adhérant au support et résistant aux conditions de lavage décrites dans l'exemple 1, avec une densité d'environ 40'000 cellules /mm².

Exemple 3

On procède comme décrit dans l'exemple 1, sauf que la suspension de cellules dans la solution aqueuse de nitrate d'aluminium est centrifugée à 2'000 g durant 5 minutes et que les cellules sont remises en suspension dans l'eau, cette suspension étant versée sur la plaque de verre.

On obtient également une couche cellulaire régulière de cellules adhérant au support et résistant aux conditions de lavage décrites dans l'exemple 1, avec une densité d'environ 40'000 cellules/mm².

Exemple 4

On mélange dans des récipients en matière plastique, par exemple du polypropylène, 20 ml d'une suspension de Saccharomyces cerevisiae à la concentration d'environ $400.10^6$ cellules/ml et 20 ml d'une solution aqueuse fraîchement préparée de nitrate ferrique à une concentration de 50 à 200 mg Fe/l.

Le pH est soit laissé tel quel, soit ajusté à 3 par $HNO_3$. Les récipients sont fermés et agités pendant l h. à température ambiante ; à ce stade les ions ferriques dissous sont des ions simples. Ensuite les suspensions sellulaires, contenant un excès d'ions, sont versées dans un récipient dans lequel se trouvent des plaques de verre. La hauteur de la solution est 1 cm au-dessus de la surface du verre. Après 4 h., moment auquel la solution contient à la fois des ions simples et des ions polynucléaires, les plaques sont lavées durant 30 minutes par un flux d'eau d'une épaisseur de 4 mm et d'une vitesse moyenne de 8 cm/ sec. Les plaques sont examinées en microscopie optique et les cellules immobilisées sont comptées.

Tableau 1

Résultats d'immobilisation sur le verre de cellules de Saccharomyces cerevisiae par traitement de celles -ci et du support par des solutions contenant des ions ferriques.

| Composition du mélange de la suspension cellulaire et de la solution ferrique | | Densité de cellules immobilisées (cellules/mm²) |
|---|---|---|
| Concentration totale en Fe (Mg/l) | pH | |
| 25 | 3,4 | 40 000 |
| 50 | 3,3 | 39 600 |
| 100 | 3,1 | 39 000 |
| 50 | ajusté à 3,0 | 38 000 |

Dans tous les cas, les cellules immobilisées sont distribuées de manière régulière, formant une couche cellulaire homogène dont la dinsité est donnée au tableau 1.

Il faut souligner qu'aucun précipité d'un composé du fer ne se forme dans ces conidtions opératoires.

Le test au bleu de méthylène indique 90 à 99 % des cellules immobilisées gardent la capacité de réduire spontanément le colorant. Les cellules immobilisées gardent la capacité de se multiplier.

## Exemple 5

On mélange dans des récipients en plastique 20 ml d'une suspension de Saccharomyces cerevisiae à la concentration d'environ $400.10^6$ cellules/ml et 20 ml d'une solution fraîchement préparée de nitrate ferrique à une concentration de 200 mg Fe/l. Le pH est ajusté à 3 par $HNO_3$. Les récipients sont fermés et agités durant 15 h. à température amibante. Ensuite la suspension cellulaire est centrifugée à 2'000 g durant 5 minutes; le surnageant est éliminé et les cellules sont remises en suspension dans l'eau. Cette suspension est versée sur des plaques de verre comme décrit dans l'exemple 4; les plaques sont lavées et examinées comme décrit dans l'exemple I.

On obtient une couche régulière unique de cellules adhérant au support, avec une densité de 35.000 cellules/mm².

Les conditions physico-chimiques (concentration, pH, âge) de la solution ferrique au contact des cellules sont telles qu'elle contient des (ions) polynucléaires. De plus, elle ne contient aucun précipité d'un composé du fer, ce qui présent l'avatage d'obtenir un dépot très régulier adhérant fortement au support.

## Exemple 6

Des plaques de verre sont immergées pendant un temps donné dans des solutions aqueuses fraîchement préparées de nitrate ferrique à 100 mg Fe/l et de pH variable. La durée de l'immersion et le pH de la solution sont donnés au tableau 2; dans tous les cas les conditions physico-chimiques (concentration, pH, âge) de la solution en fin de traitement sont telles qu'elle contient des ions polynucléaires; elle ne contient aucun précipité d'un composé du fer.

Les plaques ainsi traitées sont rincées à l'eau et placées dans un récipient dans lequel on verse 50 ml d'une suspension cellulaire de Saccharomyces cerevisiae à la concentration d'environ $250.10^6$ cellules/ml. Après 4 h. les plaques sont lavées durant 30 minutes par un flux laminaire d'eau d'une épaisseur de 4 mm et d'une vitesse moyenne de 8 cm/sec. Les plaques sont examinées en microscopie optique et les cellules immobilisées sont comptées.

Dans tous les cas, les cellules immobilisées sont distribuées de manière régulière, formant une couche cellulaire homogène dont la densité est présentées au tableau 2.

Les résultats du test au bleu de méthylène et du test de multiplication cellulaire sont les mêmes que l'exemple 4.

Tableau 2

Résultats d'immobilisation de cellules de Saccharomyces cerevisiae sur le verre par traitement préalable de support par une solution contenant des ions ferriques.

| pH de la solution | Temps de contact verre solution | Quantité fixée (cellules/mm²) |
|---|---|---|
| 2,9 à 2,7 | 48 h | 40 000 |
| ajusté à 4 | 17 h | 38 500 |
| ajusté à 4 | 1 h | 38 300 |
| ajusté à 4 | 1 min. | 37 000 |
| ajusté à 3,85 | 40 min. | 39 000 |

## Exemple 7

On mélange 20 ml d'une suspension de Saccharomyces cerevisiae avec 20 ml d'une solution aqueuse fraîchement préparée de nitrate ferrique. Les concentrations finales sont de $200.10^6$ cellules par ml et de 100 mg/l en fer. Ce mélange est agité pendant une heure à température ambiante, les cellules sont alors séparées de la solution par centrifugation à 2'000 g pendant 5 minutes, et remises en suspension dans 40 ml d'eau ou d'une solution de $HNO_3$ à pH 3.

Des plaques de verre sont immergées dans une solution aqueuse fraîchement préparée de nitrate ferrique à la concentration de 100 mg Fe/1. La durée du trempage est soit d'une heure, soit de deux jours. Ensuite les plaques sont rincées à l'eau.

Les plaques de verre humides, ainsi traitées par les ions métalliques, sont placées dans un récipient dans lequel on verse la suspension cellulaire dont les cellules ont été traitées par des ions métalliques.
On met ainsi en présence:
  — soit les plaques maintenues 2 jours au contact de la solution ferrique et les cellules traitées et remises en suspension dans l'eau
  — soit les plaques maintenues 2 jours au contact de la solution ferrique et les cellules traitées et remises en suspension à pH 3,
  — soit les plaques maintenues 1 heure au contact de la solution ferrique et les cellules traitées et remises en suspension dans l'eau.

On laisse reposer durant 4 h ; ensuite on lave les plaques durant 20 minutes par un flux laminaire d'eau de 4 mm d'épaisseur et de vitesse moyenne de 8 cm/sec. Les plaques sont examinées en microscopie optique et les cellules immobilisées sont comptées.

Dans tous les cas les cellules immobilisées sont distribuées de manière régulière, formant une couche cellulaire homogène dont la densité est d'environ 40'000 cellules/mm².

## Revendications

1. Procédé d'immobilisation de cellules microbiennes globulaires sur un support solide, caractérisé en ce que l'on traite, préalablement à l'immobilisation, les cellules et/ou le support solide par des solutions contenant des ions métalliques simples et/ou polynucléaires, par adsorption desdits ions sur les cellules et/ou le support ainsi traités, de manière à fixer une couche cellulaire unique distribuée régulièrement sur toute la surface du support.

2. Procédé suivant la revendication 1, caractérisé en ce que les solutions contenant des ions métalliques simples ou polynucléaires proviennent de la mise en solution d'un ou de plusieurs sels métalliques.

3. Procédé suivant les revendications 1 et 2, caractérisé en ce que les sels métalliques donnant naissance aux ions métaliques simples sont choisis parmi les chlorure, sulfate, nitrate, de magnésium et d'alcalinoterreux, d'aluminium, de métaux de transition.

4. Procédé suivant les revendications 1 à 3, caractérisé en ce que les sels métalliques donnant naissance aux ions métalliques polynucléaires sont choisis parmi les chlorure, sulfate, nitrate, d'aluminium et de métaux de transition.

5. Procédé suivant les revendications 1 à 4, caractérisé ce que le support solide est choisi parmi les composés minéraux et organiques.

6. Procédé suivant les revendications 1 à 5, caractérisé en ce que le support solide est un solide siliceux ou un polymère synthétique.

7. Procédé suivant les revendications 1 à 6, caractérisé en ce que les cellules microbiennes globulaires proviennent de cellules de Sccharomyces cervisiae.

8. Cellules micorbiennes globulaires immobilisées sur un support solide, obtenues suivant les procédés faisant l'objet des revendications 1 à 7.

9. Complexe stable d'une couche de cellules globulaires distribuées uniformément sur un support solide, obtenu par l'application des procédés faisant l'objet des revendications 1 à 8.

**Patensansprüche**

I. Verfahren zur Immobilisation von kugelförmigen Bakterienzellen auf einem festen Träger, dadurch gekennzeichnet, dass man vor der Immobilisation die Zellen und/oder den festen Träger mit einfachen und/oder mehrkernigen Metallionen enthaltenden Lösungen durch Adsorption dieser Ionen auf den so behandelten Zellen und/oder dem so behandelten Träger behandelt, um eine einzige, gleichmässig verteilte zellulare Schicht auf der ganzen Fläche des Trägers zu fixieren.

2. Verfahren nach Anspruch 1, dadurch gekennzeichnet, dass die einfache oder mehrkernige Metallionen enthaltenen Lösungen aus dem Inlösungbringen eines oder mehrerer Metallsalze stammen.

3. Verfahren nach den Ansprüchen 1 und 2, dadurch gekennzeichnet, dass die einfache Metallionen liefernden Metallsalze ausgewählt sind unter den Chloriden, Sulfaten, Nitraten von Magnesium und Erdalkalien, Aluminium und Übergangsmetallen.

4. Verfahren nach den Ansprüchen 1 bis 3, dadurch gekennzeichnet, dass die mehrkernige Ionen liefernden Metallsalze ausgewählt sind unter den Chloriden, Sulfaten, Nitraten von Aluminium und Übergansmetallen.

5. Verfahren nach den Ansprüchen 1 bis 4, dadurch gekennzeichnet, dass der feste Träger ausgewählt ist aus den mineralen oder organischen Verbindungen.

6. Verfahren nach den Ansprüchen 1 bis 5, dadurch gekennzeichnet, dass der Träger ein kieseliger Feststoff oder ein synthetisches Polymer ist.

7. Verfahren nach den Ansprüchen 1 bis 6, dadurch gekennzeichnet, dass die kugelförmigen Bakterienzellen von Zellen der Saccharomyces Cerevisiae stammen.

8. Kugelförmige Bakterienzellen immobilisiert auf einem festen Träger erhalten nach den Verfahren gmäss den Ansprüchen 1 bis 7.

9. Stabiler Komplex einer einzigen Schicht kugelförmiger Zellen, die gleichmässig auf einem festen Täger verteilt sind, der durch Ausübung von Verfahren gemäss den Ansprüchen 1 bis 8 erhalten ist.

**Claims**

1. Process for immobilising globular microbial cells on a solid support, charactersed in that, prior to the immobilisation, the cells and/or the solid support are treated with solutions containing simple and/or polynuclear metal ions by adsorption of said ions on the cells and/or support thus treated, so as to fix a single cellulare layer distributed evenly over the entire surface of the support.

2. Process according to Claim 1, characterised in that the solutions containing simple or polynuclear metal ions originate from the placing in solution of one or more metal salts.

3. Process according to Claims 1 and 2, characterised in the metal salts giving rise to the simple metal ions are chosen from among the chlorides, sulphates, nitrates of magnesium and alkaline earths, of aluminium, and of transition metals.

4. Process according to Claims 1 to 3, characterised in that the metal salts giving rise to the polynuclear metal ions are chosen from among the chlorides, sulphates, nitrates of aluminium and of transition metals.

5. Process according to Claims 1 to 4, characterised in that the solid support is chosen from among the mineral and organic compounds.

6. Process according to Claims 1 to 5, characterised in that the solid support is a siliceous solid or a synthetic polymer.

7. Process according to Claims 1 to 6, characterised in that the globular microbial cells originate from cells of Saccharomyces cervisiae.

8. Globular microbial cells immobilised on a solid support, obtained in accordance with the processes forming the subject-matter of Claims 1 to 7.

9. Stable complex of a single layer of globular cells distributed uniformly on a solid support, obtained by the application of the processes forming the subject-matter of Claims 1 to 8.